# EUROPEAN PATENT APPLICATION

(11) **EP 2 305 162 A1**
(43) Date of publication of application: **06.04.2011**
(21) Application number: 10181278.2
(22) Date of filing: 28.09.2010
(51) Int. Cl.: A61B 19/00

(54) **Interface for actuating a robotic manipulator from a remote position for manoeuvring steerable catheters in the human cardiovascular system**

(30) Priority: 30.09.2009 IT BO20090074 U
(71) Applicant: Tre Esse Progettazione Biomedica S.r.l, 40138 Bologna (IT)
(72) Inventor: Plicchi, Gianni, I-40125, Bologna (IT); Marcelli, Emanuela, I-40125, Bologna (IT); Cercenelli, Laura, I-40138, Bologna (IT); Panfili, Mauro, I-40139, Bologna (IT); Golinelli, Daniele, I-40131, Bologna (IT)
(74) Representative: Porsia, Dino

(57) **Abstract**

Interface for actuating a robotic manipulator (B) from a remote position for manoeuvring steerable catheters (C) in the human cardiovascular system, comprising a casing (1) with controls which are actuated by an operator in order to remotely control said manipulator, **characterized in that** these controls comprise a shaft (102) with a hand grip (2) which can be grasped by one of the operator's hands as though he were grasping the handle (D) of said catheter, the hand grip being designed to be subjected to small axial movements in both directions (X) and/or to small rotary movements in both directions (Y), and sensors being provided to detect said movements and to operate the corresponding actuators of the robotic manipulator which are responsible for the catheter's axial movement in both directions and/or for its rotation in both directions, the whole arrangement being such that the operator can control the catheter by means of said hand grip (2) as if he were grasping it directly by the end handle (D), with the resulting benefits in terms of convenience, user-friendliness and ease of use.

## Description

The invention relates to an interface for actuating a robotic manipulator from a remote position for manoeuvring temporary catheters for electrophysiology procedures in the hu>man cardiovascular system, these catheters having control systems for deflecting their distal ends and therefore being described as "steerable". The robotic manipulators in question can, for example, be of the type described in European publication EP 2 218 396 and corresponding Us publication US-2010-0204646-A1, partially illustrated in Figure 1, which uses an introduction, adaptation and positioning device A of the type protected by Italian patent 1363313 and by European publication EP 2 218 474 and corresponding US publication US-2010-0204680-A1, and which comprises a robotic apparatus B which is to be placed near the patient P, in a relatively fixed position which is predetermined by means of a suitable adjustable support H, and which supports and controls the catheter C by its handle D and comprises telescopic guiding and stiffening means E by which the portion of the catheter which is located outside the patient's body between the handle and said adaptation and introduction device A, and which is acted on by said guide means, remains essentially rigid and therefore cannot carry out bending movements required by the tip force when the catheter is manoeuvred by means of the handle D of the robotic manipulator B. The robotic manipulator is designed to support the handle D and the slider F or other suitable means for deflecting the distal end of the catheter, and can subject the catheter, by means of the handle, to axial advance and retraction movements as indicated by the double arrow X, to rotary movements in both directions as indicated by the double arrow Y, and, by the axial movement in the direction of the double arrow Z imparted to the slider F or to another control device associated with the handle, to opposed steering movements, in order to steer the tip of the catheter through the twists and turns of the patient's cardiovascular system to reach a predetermined point in this system, in a controlled, recordable and safe way.

By means of the multi-wire electrical connection indicated by B1 and/or other suitable means, the robotic manipulator B can be connected to an interface located in a remote shielded position, through which an operator can remotely control the operation of the movement means of the manipulator B, while remaining in a position protected from the ionizing radiation emitted by the system which displays the navigation of said catheter in the human cardiovascular system. The invention relates to an interface I of this kind for remotely controlling said robotic manipulator, because the technicians responsible for manually manoeuvring said catheters have found it difficult to use the interfaces devised up to the present time, which use push buttons or joystick controllers. The object of the invention is to overcome the limitations of the prior art by means of an interface according to Claim 1 and the subsequent dependent claims, based essentially on the use of a hand grip controller which can be grasped by one hand of the operator in the same way as the handle of the catheter would be grasped, this hand grip controller being capable of small axial movements in both directions and small rotary movements in both directions, and being capable of being combined with a rocker switch or equivalent type of controller, sensors being provided to detect said movements and to operate the corresponding actuators of the robotic manipulator which move the catheter axially in both directions, rotate it in both directions, and steer it in opposite directions. By using the hand grip controller of the novel interface, the operator can control the catheter as if he were grasping it directly by its end handle, thus obtaining evident benefits in terms of convenience, practicality, user-friendliness and ease of use.

Further characteristics of the invention, and the advantages derived therefrom, will be made clear by the following description which refers to the figures of the two attached sheets of drawings, in which:
- Fig. 1 is a perspective view of the interface according to the invention connected to a robotic manipulator of the aforesaid type;
- Figs. 2 and 3 are perspective views of the components of the interface, seen respectively from above and from the opposite side to the side connected to the hand grip controller.

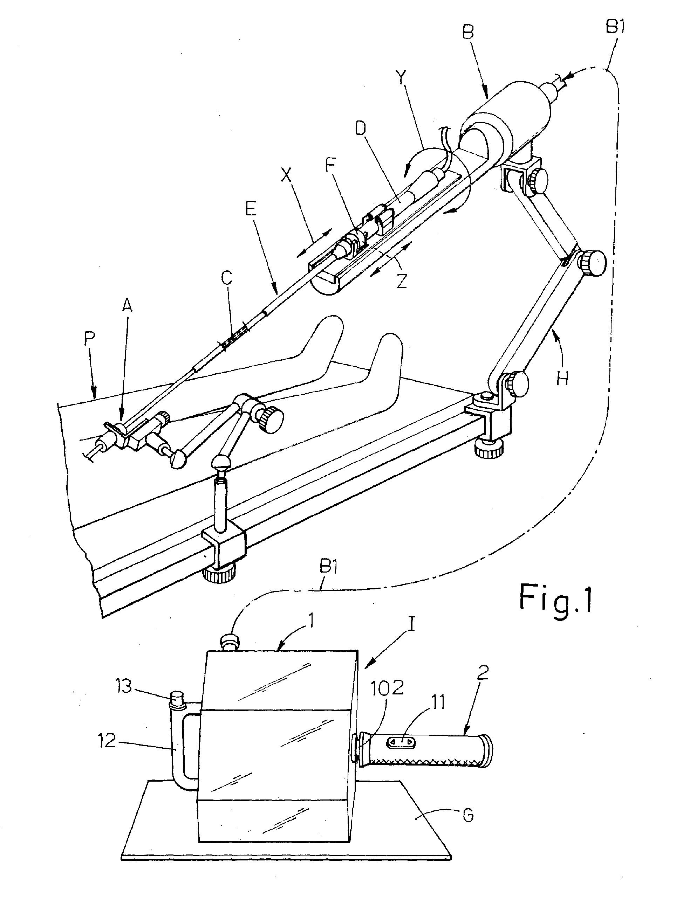

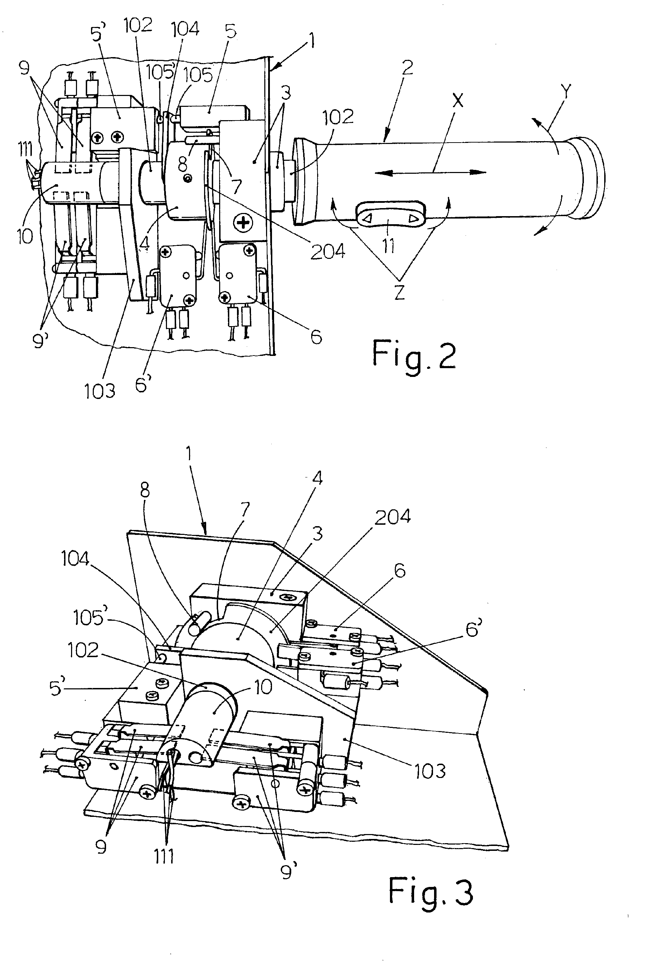

In these drawings it can be seen that the interface I comprises a casing 1 of any suitable shape, having an inclined front wall in the form of a lectern on which any necessary push buttons, controls, sensors, indicators or instruments, not shown in Figure 1, can be mounted for the uses for which the interface is intended, this casing 1 being placed in a stable position, for example by fixing it removably, using magnetic and/or other fastenings, to a large base G which rests on a surface with the interposition of an anti-slip mat, made from rubber for example. Clearly, other suitable means can be used to support the casing 1 stably in its operating position. A hand grip 2, covered with an anti-slip material and having dimensions such that it can conveniently be grasped by an operator's hand, is mounted on one side of the casing 1 so as to project horizontally therefrom. The hand grip 2 is mounted on a hollow shaft 102, supported rotatably by a support 3, made from suitable plastic material for example, fixed to a lateral aperture in the casing 1 through which a portion of said shaft of suitable length passes, this portion being rotatably supported by a further support 103 which is also fixed suitably to the casing 1. On the portion of shaft 102 lying between the supports 3 and 103, a sleeve 4, made from plastic or other suitable material for example, is axially fixed and keyed, this sleeve having a flange sector 104 fixed on its end facing the support 103, the flange being formed in one piece with the sleeve or applied thereto, and having the rounded ends of pins 105, 105' acting on its opposite faces, these pins being parallel to the shaft 102 and axially slidable in corresponding guide and limit blocks 5, 5' which are fixed to the casing 1 and which house resilient means which push said pins outwards with a substantially equal force, in such a way that the shaft 102 and the hand grip 2 are kept stably in a predetermined axial position and can be moved axially in both directions through predetermined distances which depend on the distance between said blocks 5 and 5', as indicated by the double arrow X in Figure 2. These axial movements are detected by sensors or microswitches 6 and 6' which are fixed inside the casing, are preferably duplicated to provide a redundant and secure circuit, and have moving parts which interact with the opposite faces of a flange 204 of said sleeve 4.

The flange 204 has a perimetric opening 7 of suitable angular amplitude through which a pin 8 fixed to the adjacent support 3 passes, the whole arrangement being such that, because of the presence of these means, the hand grip 2 and the shaft 102 can be rotated by the operator in opposite directions and with a predetermined angular movement, as indicated by the double arrow Y in Figure 2. This rotary movement of the hand grip 2 is detected by pairs of sensors or microswitches 9 and 9' whose bodies are fixed in the casing 1 and whose sprung moving parts interact with the opposite sides of a cam 10 fixed to the end of the shaft 102 located inside the casing 1, in such a way that the assembly consisting of the cam, the shaft and the hand grip 2 is also stabilized in a predetermined angular position. Finally, it can seen in Figure 2 that a double-acting rocker switch 11 is mounted on the hand grip 2 in a housing formed in the hand grip itself and also in a corresponding underlying portion of the shaft 102, in a position close to the support 3, this switch being such that it can be conveniently rocked in the two directions indicated by the arrows Z by the operator's hand which is also grasping the hand grip 2, and being connected to wires 111 which pass through the hollow shaft 102 and through the cam 10 which is also hollow, and which emerge from the free end of the cam as shown clearly in Figure 3.

The wires connected to the microswitches 6, 6', 9, 9' and the wires 111 from the switch 11 are connected, by means of the multi-wire connection B1 of Figure 1 and/or other suitable means, to the robotic manipulator B which moves the handle D and the slider F of the catheter in the aforesaid ways as indicated by the corresponding arrows X, Y and Z.

The switch 11 is relatively wide, and can therefore always be correctly orientated with part of the switch facing the operator, for convenient operation. However, it is to be understood that the switch 11 can be replaced by a different double-acting switch, such as a slider switch, with the moving part in the form of an annular sector, thus simulating in all respects the slider F of the catheter. It will be clear from the above description that the interface in question is highly convenient and simple to use by personnel having the normal degree of specialist training in the introduction of steering catheters C into the human cardiovascular system, since the hand grip controller 2 of the interface I simulates in all respects the handle D of a catheter.

The robotic manipulator B to which reference has been made is provided with means for detecting the catheter tip force and for triggering an alarm signal if this force exceeds specified levels. The interface I can be provided with any suitable means (not shown) for emitting what is known as an alarm signal, for example in the form of sounds, vibrations and/or indicator lamps, and/or can be provided with means for automatically modifying the force of the resilient means which oppose the movements of the hand grip 2, so as to transmit sensations of variable resistance to the operator in order to help him to move the catheter in the patient correctly and in a safe manner by means of the robotic manipulator B. Instead of the sensors or microswitches and rocker switch described, it is possible to use means capable of providing progressive and proportional control of the movement imparted to them and capable of controlling the motors of the robotic manipulator B at a variable speed which is proportional to said movements.

In a preferred embodiment of the invention, the interface includes a safety push button or other control device 13, which is mounted in any suitable position on the casing 1 or on parts associated therewith, can be operated by the hand of the operator not grasping the hand grip 2, and can enable or disable any command imparted to the robotic manipulator B through the hand grip 2 or through other control means. Finally, in order to stabilize the interface when in use and/or for other reasons, the casing 1 can be provided with an auxiliary handle or grip 12, which can be placed opposite the hand grip 2 as shown in the example in Figure 1, and can be grasped by the operator with the hand which is not used to grasp the hand grip 2. Said safety push button or control device 13 can advantageously be placed on this auxiliary handle or grip 12.

## Claims

1. Interface for actuating a robotic manipulator (B) from a remote position for manoeuvring catheters (C) in the human cardiovascular system, comprising a casing (1) with controls which are actuated by an operator in order to remotely control said manipulator, **characterized in that** these controls comprise a shaft (102) with a hand grip (2) which can be grasped by one of the operator's hands as though he were grasping the handle (D) of said catheter, the hand grip being designed to be subjected to small axial movements in both directions (X) and/or to small rotary movements in both directions (Y), and sensors being provided to detect said movements and to operate the corresponding actuators of the robotic manipulator which are responsible for the catheter's axial movement in both directions and/or for its rotation in both directions, the whole arrangement being such that the operator can control the catheter by means of said hand grip (2) as if he were grasping it directly by the end handle (D), with the resulting benefits in terms of convenience, user-friendliness and ease of use.

2. Interface according to Claim 1, in which said hand grip (2) is associated with a rocker switch (11) or other type of double-acting switch, which can be operated by the hand with which the operator grasps the hand grip, and which is connected electrically to the actuator of the robotic manipulator (B) which is responsible for the steering in opposite directions (Z) of the catheter if it is of the steerable type.

3. Interface according to Claim 1, in which said casing (1) contains resilient and limiting means (5, 105, 5', 105') for stabilizing the hand grip (2) in a predetermined axial rest position and for allowing it to move axially in both directions over a small predetermined distance which is sufficient for the operation of corresponding microswitches or sensors (6, 6'), and which is also **characterized in that** it comprises means (7, 8) for allowing a small angular rotation of the hand grip in both directions, sufficient for the operation of corresponding microswitches or sensors (9, 9'), resilient means being provided to keep the hand grip in a predetermined angular rest position.

4. Interface according to any one or more of the preceding claims, in which said hand grip (2) is placed horizontally and projects from the casing (1) which contains the various controls for the remote control of the robotic manipulator, is covered laterally with a rubberized anti-slip surface and has said double-acting push button or control device (11) mounted on it to activate the catheter steering movement, this push button or control device being located in the vicinity of the end of the hand grip that is close to the casing (1) and being wide enough to be in a useful and convenient position for operation, throughout the angular movement which can be imparted to said hand grip.

5. Interface according to any one or more of the preceding claims, in which at least said microswitches or sensors (6, 6', 9, 9') are duplicated to form a redundant and secure control circuit.

6. Interface according to any one or more of the preceding claims, in which said microswitches or sensors (6, 6', 9, 9') and said double-acting switch (11) are designed to generate an ON-OFF command or a command proportional to the movement imparted to it, which is converted by other suitable means into a command for the operation at variable speeds of the internal actuators of the robotic manipulator.

7. Interface according to any one or more of the preceding claims, **characterized in that** it comprises means to indicate to the operator any variations in the catheter tip force during introduction into the human cardiovascular system, these signals being indicated in the form of sound or light or being converted by suitable means into a proportional variation of the resistance which the operator encounters in actuating said hand grip (2) and its associated controls.

8. Interface according to any one or more of the preceding claims, **characterized in that** a safety push button or control device (13) can be placed in any suitable position on the casing (1) or on parts associated with it, in such a way that it can be activated by the operator's hand that is not grasping the hand grip (2), for enabling or disabling any command imparted to the robotic manipulator (B) by means of the hand grip (2) or other means.

9. Interface according to any one or more of the preceding claims, in which the casing (1) can be provided, in a position opposite that of the hand grip (2) for example, with an auxiliary handle (12) which can be grasped by the operator's hand which is not grasping said hand grip (2), and said safety control device or push button (13) can advantageously be placed on or connected to this handle (12).
